# EUROPEAN PATENT SPECIFICATION

(11) **EP 1 915 147 B1**
(45) Date of publication and mention of the grant of the patent: **24.04.2013**
(21) Application number: 06754706.7
(22) Date of filing: 10.07.2006
(51) Int. Cl.: A61K 31/4045, A61K 31/4035, A61K 31/4439, A61K 31/47, A61K 31/4709, A61K 31/165, A61K 31/27, A61P 1/04, A61P 13/00

(54) **USE OF MGLUR5 (ESP. AFQ056) IN GI (ESP. GERD)**
VERWENDUNG VON MGLUR5 (ESP. AFQ056) BEI GI (ESP. GERD)
EMPLOI DE MGLUR5 (EN PARTICULIER AFQ056) DANS LE TRACTUS GASTRO-INTESTINAL (EN PARTICULIER DANS LE CAS DE REFLUX GASTRO-OESOPHAGIEN)

(30) Priority: 12.07.2005 GB 0514296
(43) Date of publication of application: 30.04.2008
(73) Proprietor: Novartis AG, 4056 Basel (CH)
(72) Inventor: ROUZADE-DOMINGUEZ, Marie-Laure, F-68480 Werentzhouse (FR); PFANNKUCHE, Hans-Jürgen, 79576 Weil (DE); GASPARINI, Fabrizio, CH-4415 Lausen (CH)
(74) Representative: Jeffries, Charles Edward
(86) International application number: PCT/EP2006/006729
(87) International publication number: WO 2007/006530

(56) References cited:
- WO-A-03/047581
- WO-A-2004/000316
- WO-A-2004/067002
- US-A1- 2005 143 375

## Description

The present invention relates to new pharmaceutical uses of compounds acting as antagonists of metabotropic glutamate type-5 receptors ("mGluR5 antagonists"). The pharmaceutical uses include the treatment, prevention and/or delay of progression of gastro-esophageal reflux disease.

WO 03/047581 discloses mGluR5 antagonists and their use as pharmaceuticals, especially in the treatment of nervous system disorders. WO 05/044265, WO 05/044266, WO 05/044267 disclose mGluR5 antagonists and their use as pharmaceuticals, especially in the treatment or prevention of gastro-esophageal reflux disease (GERD).

The known medication has some drawbacks in terms of limited efficacy, tolerability, convenience, and safety.

It has surprisingly found that the compound (-)-(3aR,4S,7aR)-4-Hydroxy-4-m-tolylethynyl-octahydro-indole-1- carboxylic acid methyl ester is highly effective in the treatment, prevention and/or delay of progression of disorders of the gastro-intestinal and urinary tract.

Gastro-Esophageal Reflux Disease (GERD) results from the retrograde flow of gastric contents into the esophagus. It is the most common ailment in the upper gastro-intestinal tract; its cardinal feature and symptom is commonly known as "heartburn". A major factor considered for GERD is an incompetence of the Lower Esophageal Sphincter that opens transiently and allows passage of material (e.g. meal, acidic fluid or bile), from the stomach into the esophagus. This motor event denominated Transient Lower Esophageal Sphincter Relaxation (TLESR) occurs more often in patients suffering from GERD than in healthy subjects and occurs more often in infants with regurgitation. Current standard therapies in GERD aim at suppressing gastric acid secretion or enhancing gastrointestinal motility to limit the exposure of the esophagus to acidic gastric contents. Frequent exposure of the esophageal mucosa to acid can trigger pain (often perceived as heartburn) and lead to erosions. It can also lead to extra-esophageal disorders such as asthma, cough and laryngitis. Todate, there is no treatment available which reduces the occurrence of TLESRs and, thereby, the symptoms associated with GERD or regurgitation in infants.

Pain and/or Discomfort is often associated with FGIDs, disorders of the .urinary tract and post-operative ileus; it is not only a symptom of GERD. Patients suffering from Irritable Bowel Syndrome (IBS), dyspepsia, diseases of the biliary tract, pancreas, urinary bladder and post-operative conditions report pain and discomfort. Visceral hypersensitivity has been discovered as a key phenomenon in many patients suffering from conditions like IBS, dyspepsia, GERD, functional heartburn and other conditions listed above. To date, there is no medication available which specifically treats visceral hypersensitivity and, thereby, reduces symptoms of pain/discomfort in patients suffering from GERD, functional heartburn, IBS, dyspepsia, diseases of the biliary tract, pancreas, urinary bladder and post-operative conditions. Pain, as used in this specification, includes visceral pain and/or visceral discomfort.

The present invention accordingly provides the use of the compound for treatment of GERD.

The present invention accordingly provides the use of the compound for prevention of GERD.

The present invention accordingly provides the use of the compound for delay of progression of GERD.

A further aspect of the invention relates to the use of the compound for the manufacture of a medicament for the treatment of GERD.

A further aspect of the invention relates to Pharmaceutical composition comprising the compound according to claim 1 for the treatment, prevention and / or delay of progression of GERD

For the above-mentioned indication (the condition disorder) the appropriate dosage will vary depending upon, for example, the compound employed, the host, the mode of administration and the nature and severity of the condition being treated. However, in general, satisfactory results in animals are indicated to be obtained at a daily dosage of from about 0.01 to about 100 mg/kg body weight, preferably from about 0.1 to about 10 mg/kg body weight, e.g. 1 mg/kg. In larger mammals, for example humans, an indicated daily dosage is in the range from about 0.1 to about 1000 mg, preferably from about 1 to about 400 mg, most preferably from about 10 to about 100 mg of the compound conveniently administered, for example, in divided doses up to four times a day.

For use according to the invention, the compound may be administered as single active agent or in combination with other active agents, in any usual manner, *e.g*. orally, for example in the form of tablets or capsules, or parenterally, for example in the form of injection solutions or suspensions.

Moreover, the present invention provides a pharmaceutical composition comprising the compound in association with at least one pharmaceutical carrier or diluent for use in the treatment of GERD. Such compositions may be manufactured in conventional manner. Unit dosage forms may contain, for example, from about 2.5 to about 25 mg of the compound of formula (I).

The present invention also provides the use of the compound for the manufacture of a pharmaceutical composition for the treatment of the above-indicated disease / condition.

The effectiveness of the compounds can be shown by a number of well established tests / models, including but not limiting to a GERD model in dogs, a model of fasted gastric tone and gastric accommodation to meal in dogs.

### GERD model in dogs:

Beagle dogs are equipped with a chronic esophagostomy to allow passage of a manometric catheter and a pH probe along the esophagus and the stomach. Following recording of basal pressures of the Lower Esophageal Sphincter and the stomach, one of the novel compound of formula (I) described in WO 03/047581 A1 is administered at doses of 0.03, 0.1, 0.3 and 1 mg/kg i.v.. Transient Lower Esophageal Sphincter Relaxations (TLESRs) and acid reflux are induced by infusion of an acidified meal followed by stomach distention using a peristaltic pump infusing air at 40 ml/min, according to a modification of Stakeberg, J. and Lehmann, A. Neurogastroenterol. Mot. (1999) 11: 125-132. Selected compounds of formula (I) reduce dose-dependently the frequency of TLESRs and TLESRs associated with acid reflux. The extent of inhibition obtained for TLESRs as compared to vehicle control was 37 % (4.0 ± 0.5, n = 6 versus vehicle control 6.4 ± 0.6, n = 10, P < 0.05) and 62 % (2.4 ± 0.2, n = 5 versus vehicle control 6.4 ± 0.6, n = 10, P < 0.01) at doses of 0.3 and 1 mg/kg i.v., respectively. For TLESRs with acid reflux the extent of inhibition as compared to vehicle control was 85 % (0.3 ± 0.2, n = 6 versus vehicle control 2.1 ± 0.3, n = 10, P < 0.05) and 70 % (0.6 ± 0.4, n = 5 versus vehicle control 2.1 ± 0.3, n = 10, P > 0.05) at doses of 0.3 and 1 mg/kg i.v., respectively

### Model of fasted gastric tone and gastric accommodation to meal in dogs:

Beagle dogs are equipped with a chronic esophagostomy to allow passage of a balloon (10 cm long, max. volume 950 ml) into the stomach. Approx. 200 ml of air is slowly infused to unfold the balloon, the catheter is retracted to adequately position the balloon in the proximal stomach. After deflation of the balloon, the dog is left undisturbed for 30 min to allow the stomach to return to fasting volume. The balloon is then connected to a barostat device controlling both pressure and volume of the balloon. The minimal distending pressure (MDP) is determined and the balloon pressure is then set at MDP + 2 mmHg for 60 min. Afterwards, the pressure is lowered to 0 mmHg while a liquid nutritive meal (20 ml/kg body weight) is infused intragastrically at a speed of 100 ml/min. The balloon is re-inflated at a pressure of MDP + 2 mmHg for 60 min while meal - induced gastric accommodation occurs. The compound of formula (I) at a dose of 3 mg/kg or its vehicle is administered orally 10 min after the first balloon distension in the fasting condition. Baseline fasting gastric volume prior to administration was set at 100 %. The fasting gastric volume averaged every 10 min (from 0 to 50 min) post vehicle administration amounted to 85 %, 81 %, 79 %, 69 % and 68 %, respectively. In comparison, a selected compound of formula (I) increased the fasting volume; the averaged volumes (from 0 to 50 min) amounted to 97 %, 97 %, 159 %, 188 % and 197 %, respectively. One-way repeated measure ANOVA revealed that the gastric volume decreased significantly post vehicle administration (n = 5, P < 0.01), however, it increased significantly post administration of selected compound of Formula (I) (n = 5, P < 0.01). Following administration of the meal, gastric volumes were increased significantly to 427 % (P < 0.05) and 338 % (P < 0.05) of baseline gastric volume with vehicle and,selected compound of formula (I), respectively. The results of this study show that the selected compound of formula (I) relaxes the fasted stomach (prior to meal intake) without affecting gastric accommodation as consequence of meal intake. In conclusion, selected compound of formula (I) should be useful for the treatment of dyspeptic symptoms in patients suffering from Functional Dyspepsia, GERD, Functional heartburn and IBS.

## Claims

1. The use of (-)-(3aR,4S,7aR)-4-hydroxy-4-m-tolylethynyl-octahydro-indole-1-carboxylic acid methyl ester for the manufacture of a pharmaceutical composition for the treatment, prevention and/or delay of progression of the gastro-esophageal reflux disease.

2. The use according to claim 1, wherein the pharmaceutical composition for the treatment, prevention and/or delay of progression of the gastro-esophageal reflux disease further comprises at least one pharmaceutical carrier or diluent in a unit dosage form containing from 2.5 mg to 25 mg of (-)-(3aR,4S,7aR)-4-hydroxy-4-m-tolylethynyl-octahydro-indole-1-carboxylic acid methyl ester.

## Patentansprüche

1. Verwendung von (-)-(3aR, 4S, 7aR)-4-Hydroxy-4-m-tolylethinyl-octahydro-indol-1-carbonsäuremethylester zur Herstellung einer pharmazeutischen Zusammensetzung für die Behandlung, Vorbeugung und/oder Verlangsamung des Fortschreitens von Refluxösophagitis.

2. Verwendung nach Anspruch 1, wobei die pharmazeutische Zusammensetzung für die Behandlung, Vorbeugung und/oder Verlangsamung des Fortschreitens von Refluxösophagitis weiterhin wenigstens einen pharmazeutischen Träger oder ein pharmazeutisches Verdünnungsmittel in einer Einheitsdosierungsform umfasst, welche von 2,5 mg bis 25 mg (-)-(3aR, 4S, 7aR)-4-Hydroxy-4-m-tolylethinyl-octahydro-indol-1-carbonsäure-methylester enthält.

## Revendications

1. Utilisation de l'ester méthylique d'acide (-)-(3aR,4S,7aR)-4-hydroxy-4-m-tolyléthynyl-octahydro-indole-1-carboxylique pour la fabrication d'une composition pharmaceutique destinée au traitement, à la prévention et/ou à retarder la progression du reflux gastro-oesophagien pathologique.

2. Utilisation selon la revendication 1, où la composition pharmaceutique destinée au traitement, à la prévention et/ou à retarder la progression du reflux gastro-oesophagien pathologique comprend en outre au moins un véhicule ou diluant pharmaceutique dans une forme posologique unitaire contenant de 2,5 mg à 25 mg d'ester méthylique d'acide (-)-(3aR,4S,7aR)-4-hydroxy-4-m-tolyléthynyl-octahydro-indole-1-carboxylique.
